# EUROPEAN PATENT SPECIFICATION

(11) **EP 4 079 348 B1**
(45) Date of publication and mention of the grant of the patent: **02.04.2025**
(21) Application number: 22169686.7
(22) Date of filing: 25.04.2022
(51) Int. Cl.: A61M 5/142, A61M 5/168, A61M 5/172

(54) **DISPENSING DEVICE FOR WEARABLE DRUG DELIVERY DEVICE**
ABGABEVORRICHTUNG FÜR EINE TRAGBARE ARZNEIMITTELABGABEVORRICHTUNG
DISPOSITIF DE DISTRIBUTION POUR DISPOSITIF D'ADMINISTRATION DE MÉDICAMENT PORTABLE

(30) Priority: 23.04.2021 US 202163178607 P
(43) Date of publication of application: 26.10.2022
(73) Proprietor: Insulet Corporation, Acton, MA 01720 (US)
(72) Inventor: STOKES, Jeffrey H., Harvard, 01451 (US)
(74) Representative: Peterreins Schley

(56) References cited:
- US-A1- 2012 209 207

## Description

### TECHNICAL FIELD

The disclosed embodiments generally relate to medication delivery. More particularly, the disclosed embodiments relate to techniques, processes, systems, and dispensing devices for providing a fixed volume of fluid, which is delivered and refilled within one pumping cycle.

### BACKGROUND

Many wearable drug delivery devices include a reservoir for storing a liquid drug. A drive mechanism is operated to expel the stored liquid drug from the reservoir for delivery to a user. Some conventional drive mechanisms use a plunger to expel the liquid drug from the reservoir. Accordingly, the drive mechanism generally has a length equal to a length of the reservoir. And when the reservoir is filled, these wearable drive mechanisms require a length of the drug delivery devices to be significantly larger, for example, about twice the length of the reservoir when the plunger has yet to traverse the length of the reservoir to expel fluid. Increasing the size of the drug delivery devices to accommodate filled reservoirs or pre-filled cartridges and corresponding drive mechanism components leads to bulky devices that are uncomfortable for the user to wear.

Patent application document US 2012/209207 A1 discloses a prior art pumping mechanism employing a bell crank and combining a valving pump with a flow biasing valve.

Accordingly, there is a need for a simplified system for accurately expelling a liquid drug from a reservoir, which also reduces drug delivery device size.

### SUMMARY

This Summary is provided to introduce a selection of concepts in a simplified form that are further described below in the Detailed Description. This Summary is not intended to identify key features or essential features of the claimed subject matter, nor is it intended as an aid in determining the scope of the claimed subject matter.

In some approaches, a wearable drug delivery device may include a wearable drug delivery device having a reservoir configured to store a liquid drug, and a delivery pump device including a drive mechanism coupled to the reservoir for receiving the liquid drug. The drive mechanism may include a housing having a pump chamber fluidly connected with a channel chamber, an inlet channel operable to deliver the liquid drug from the reservoir to the channel chamber, and an outlet channel operable to expel the liquid drug from the channel chamber. The drive mechanism may further include a resilient sealing member sealing the pump chamber, and a plunger adjacent the resilient sealing member, wherein the plunger is operable to enter the pump chamber to displace the liquid drug from the pump chamber.

In some approaches, a drive mechanism of a wearable drug delivery device may include a housing having a pump chamber fluidly connected with a channel chamber, an inlet channel operable to deliver a liquid drug from a reservoir of the wearable drug delivery device to the channel chamber, and an outlet channel operable to expel the liquid drug from the channel chamber. The drive mechanism may further include a resilient sealing member sealing the pump chamber, and a plunger adjacent the resilient sealing member, wherein the plunger is operable to enter the pump chamber to expel the liquid drug from the pump chamber.

Furthermore, in some approaches, a method may include coupling a drive mechanism to a reservoir configured to store a liquid drug, the drive mechanism including a housing having a pump chamber fluidly connected with a channel chamber, an inlet channel operable to deliver the liquid drug from the reservoir to the channel chamber, and an outlet channel operable to expel the liquid drug from the channel chamber. The drive mechanism may further include a resilient sealing member sealing the pump chamber, and a plunger adjacent the resilient sealing member. The method may further include biasing the plunger into the pump chamber to displace the liquid drug from the pump chamber.

### BRIEF DESCRIPTION OF THE DRAWINGS

In the drawings, like reference characters generally refer to the same parts throughout the different views. In the following description, various embodiments of the present disclosure are described with reference to the following drawings, in which:
**FIG. 1** illustrates a schematic diagram of a drug delivery system according to embodiments of the present disclosure;
**FIG. 2** illustrates a side cross-sectional view of a drive mechanism of a delivery pump device according to embodiments of the present disclosure;
**FIGs. 3A-3D** illustrate side cross-sectional views of a drive mechanism of a delivery pump device according to embodiments of the present disclosure;
**FIGs. 4A-4C** illustrate side cross-sectional views of a drive mechanism of a delivery pump device according to embodiments of the present disclosure; and
**FIG. 5** illustrates a process flow of a method according to embodiments of the present disclosure.

The drawings are not necessarily to scale. The drawings are merely representations, not intended to portray specific parameters of the disclosure. The drawings are intended to depict exemplary embodiments of the disclosure, and therefore are not be considered as limiting in scope. Furthermore, certain elements in some of the figures may be omitted, or illustrated not-to-scale, for illustrative clarity. Still furthermore, for clarity, some reference numbers may be omitted in certain drawings.

### DETAILED DESCRIPTION

Systems, devices, and methods in accordance with the present disclosure will now be described more fully hereinafter with reference to the accompanying drawings, where one or more embodiments are shown. The systems, devices, and methods may be embodied in many different forms and are not to be construed as being limited to the embodiments set forth herein. Instead, these embodiments are provided so the disclosure will be thorough and complete, and will fully convey the scope of methods and devices to those skilled in the art. Each of the systems, devices, and methods disclosed herein provides one or more advantages over conventional systems, components, and methods.

Embodiments of the present disclosure provide a drive mechanism of a delivery pump device, the drive mechanism including a pump chamber, sealed with a resilient sealing member, and a complimentary shaped plunger operable to be inserted and withdrawn from the pump chamber. When the plunger is moved into the pump chamber, the resilient sealing member deflects and fills the pump chamber completely, thus displacing a liquid drug therefrom. In some embodiments, the liquid drug is provided to a cannula or microneedle array, e.g., via an outlet channel of a housing of the drive mechanism. The volume of the pump chamber may determine the amount of liquid drug dispensed to a patient. As the plunger is moved out of the pump chamber, the liquid drug may be drawn through an inlet channel from a reservoir of the delivery pump device. In some embodiments, aspiration of the pump chamber is facilitated by the elastomeric properties of the resilient sealing member wanting to return to its natural/original shape.

In some embodiments, the inlet and outlet channels may be fluidly connected by a channel chamber. More specifically, the inlet channel may include an inlet chamber and the outlet channel may include an outlet chamber, wherein the channel chamber extends between the inlet chamber and the outlet chamber. In some embodiments, the inlet chamber and the outlet chamber are sealed by one or more resilient sealing members (e.g., a gasket). The gasket may span both the inlet and outlet chambers, allowing the inlet channel to be blocked but allowing fluid to connect from the pump chamber to the outlet chamber. Conversely, the outlet channel can be blocked by the gasket while the inlet channel is connected to the pump chamber.

In some embodiments, the drive mechanism may include a follower coupled to the housing, wherein the follower is moveable relative to the housing to control movement of the plunger. The follower may be moved in one direction by a shape memory alloy (SMA) wire and biased in a second direction by a return spring. Upon activation/contraction of the SMA wire, the follower is brought into contact with the plunger, thus forcing the plunger towards the resilient sealing member. The SMA wire may continue to contract until a tip of the plunger and the resilient sealing member are fully inserted within the pump chamber, and a fixed volume of the liquid drug is forced through the outlet channel. The fixed volume may be a function of internal chamber geometries and SMA stroke.

When the SMA wire is deactivated, the SMA wire will start to relax and the stored energy of the return spring will cause the follower to spring back to an original position. The motion of the follower in turn causes the plunger and the resilient sealing member to move out of the pump chamber, thus creating a negative pressure differential in the pump chamber and the inlet channel, which causes the liquid drug to be drawn back into the pump chamber. The cycle is then repeated by activating the SMA wire. Advantageously, the delivery pump device of the present disclosure enables a fixed volume of fluid to be delivered and refilled without any secondary steps or additional components. Said another way, the system design and material properties of the SMA wire and the resilient sealing member dictate the fluid response into and out of the pump chamber.

In various embodiments, the wearable drug delivery device described herein may include an analyte sensor, such as a blood glucose sensor, and the cannula or microneedle array may be operable in allowing the device to measure an analyte level in a user of the device.

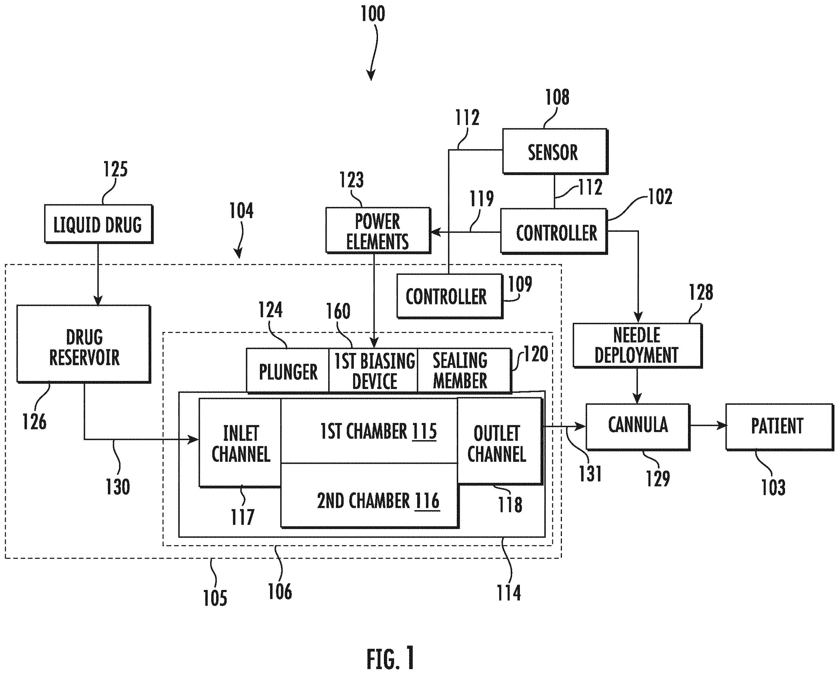

**FIG. 1** illustrates a simplified block diagram of an example system 100. The system 100 may be a wearable or on-body drug delivery device and/or an analyte sensor attached to the skin of a patient 103. The system 100 may include a controller 102, a pump mechanism 104 (hereinafter "pump 104"), and a sensor 108. The sensor 108 may be a glucose or other analyte monitor such as, for example, a continuous glucose monitor, and may be incorporated into the wearable device. The sensor 108 may, for example, be operable to measure blood glucose (BG) values of a user to generate a measured BG level signal 112. The controller 102, the pump 104, and the sensor 108 may be communicatively coupled to one another via a wired or wireless communication path. For example, each of the controller 102, the pump 104 and the sensor 108 may be equipped with a wireless radio frequency transceiver operable to communicate via one or more communication protocols, such as Bluetooth^{®}, or the like. As will be described in greater detail herein, the system 100 may also include a delivery pump device (hereinafter "device") 105, which includes a drive mechanism 106 having at least one housing 114 defining a pump chamber 115, a channel chamber 116, an inlet channel 117, and an outlet channel 118. The drive mechanism 106 may further include a resilient sealing member 120 enclosing the pump chamber 115, and a first biasing device 160 (e.g., SMA wire) operable to bias or move a plunger 124 relative to the pump chamber 115, as will be described in greater detail herein. The system 100 may include additional components not shown or described for the sake of brevity.

The controller 102 may receive a desired BG level signal, which may be a first signal, indicating a desired BG level or range for the patient 103. The desired BG level may be stored in memory of a controller 109 on device 105, received from a user interface to the controller 102, or another device, or by an algorithm within controller 109 (or controller 102) that automatically determines a BG level for the patient 103. The sensor 108 may be coupled to the patient 103 and operable to measure an approximate value of a BG level of the user. In response to the measured BG level or value, the sensor 108 may generate a signal indicating the measured BG value. As shown in the example, the controller 102 may also receive from the sensor 108 via a communication path, the measured BG level signal 112, which may be a second signal.

Based on the desired BG level and the measured BG level signal 112, the controller 102 or controller 109 may generate one or more control signals for directing operation of the pump 104. For example, one control signal 119 from the controller 102 or controller 109 may cause the pump 104 to turn on, or activate one or more power elements 123 operably connected with the device 105. As will be described in greater detail herein, in the case where the first biasing device 160 is an SMA wire, activation of the SMA wire by the power element 123 may cause the SMA wire to change shape and/or length, which in turn will change a configuration of the plunger 124 and the resilient sealing member 120. The specified amount of a liquid drug 125 (e.g., insulin, GLP-1, or a co-formulation of insulin and GLP-1; a chemotherapy drug; a blood thinner; or a pain medication) may then be drawn into the pump chamber 115, through the inlet channel 117, in response to a change in pressure due to the change in configuration of the resilient sealing member 120 and the plunger 124. Ideally, the specified amount of the liquid drug 125 may be determined based on a difference between the desired BG level and the actual BG level signal 112. The specified amount of the liquid drug 125 may be determined as an appropriate amount of insulin to drive the measured BG level of the user to the desired BG level. Based on operation of the pump 104, as determined by the control signal 119, the patient 103 may receive the liquid drug from a reservoir 126. The system 100 may operate as a closed-loop system, an open-loop system, or as a hybrid system. In an exemplary closed-loop system, the controller 109 may direct operation of the device 105 without input from the user or controller 102, and may receive BG level signal 112 from the sensor 108. The sensor 108 may be housed within the device 105 or may be housed in a separate device and communicate wirelessly directly with the device 105.

As further shown, the system 100 may include a needle deployment component 128 in communication with the controller 102 or the controller 109. The needle deployment component 128 may include a needle and/or cannula 129 deployable into the patient 103 and may have one or more lumens and one or more holes at a distal end thereof. The cannula 129 may form a portion of a fluid path coupling the patient 103 to the reservoir 126. More specifically, the inlet channel 117 may be coupled to the reservoir 126 by a first fluid path component 130. The first fluid path component 130 may be of any size and shape and may be made from any material. The first fluid path component 130 can allow fluid, such as the liquid drug 125 in the reservoir 126, to be transferred to the device 105 through the inlet channel 117.

As further shown, the outlet channel 118 may be coupled to the cannula 129 by a second fluid path component 131. The second fluid path component 131 may be of any size and shape and may be made from any material. The second fluid path component 131 may be connected to the cannula 129 to allow fluid expelled from the device 105 to be provided to the patient 103. The first and second fluid path components 130 and 131 may be rigid or flexible.

The controller 102/109 may be implemented in hardware, software, or any combination thereof. The controller 102/109 may, for example, be a processor, a logic circuit or a microcontroller coupled to a memory. The controller 102/109 may maintain a date and time as well as other functions (e.g., calculations or the like) performed by processors. The controller 102/109 may be operable to execute an artificial pancreas (AP) algorithm stored in memory (not shown) that enables the controller 102/109 to direct operation of the pump 104. For example, the controller 102/109 may be operable to receive an input from the sensor 108, wherein the input comprises analyte level data, such as blood glucose data or levels over time. Based on the analyte level data, the controller 102/109 may modify the behavior of the pump 104 and resulting amount of the liquid drug 125 to be delivered to the patient 103 via the device 105.

In some embodiments, the sensor 108 may be, for example, a continuous glucose monitor (CGM). The sensor 108 may be physically separate from the pump 104, or may be an integrated component within a same housing thereof. The sensor 108 may provide the controller 102 with data indicative of measured or detected blood glucose levels of the user.

The power element 123 may be a battery, a piezoelectric device, or the like, for supplying electrical power to the device 105. In other embodiments, the power element 123, or an additional power source (not shown), may also supply power to other components of the pump 104, such as the controller 102, memory, the sensor 108, and/or the needle deployment component 128.

In an example, the sensor 108 may be a device communicatively coupled to the controller 102 and may be operable to measure a blood glucose value at a predetermined time interval, such as approximately every 5 minutes, 1 minute, or the like. The sensor 108 may provide a number of blood glucose measurement values to the AP application.

In some embodiments, the pump 104, when operating in a normal mode of operation, provides insulin stored in the reservoir 126 to the patient 103 based on information (e.g., blood glucose measurement values, target blood glucose values, insulin on board, prior insulin deliveries, time of day, day of the week, inputs from an inertial measurement unit, global positioning system-enabled devices, Wi-Fi-enabled devices, or the like) provided by the sensor 108 or other functional elements of the system 100 or pump 104. For example, the pump 104 may contain analog and/or digital circuitry that may be implemented as the controller 102/109 for controlling the delivery of the drug or therapeutic agent. The circuitry used to implement the controller 102/109 may include discrete, specialized logic and/or components, an application-specific integrated circuit, a microcontroller or processor that executes software instructions, firmware, programming instructions or programming code enabling, for example, an AP application stored in memory, or any combination thereof. For example, the controller 102/109 may execute a control algorithm and other programming code that may make the controller 102/109 operable to cause the pump to deliver doses of the drug or therapeutic agent to a user at predetermined intervals or as needed to bring blood glucose measurement values to a target blood glucose value. The size and/or timing of the doses may be pre-programmed, for example, into the AP application by the patient 103 or by a third party (such as a health care provider, a parent or guardian, a manufacturer of the wearable drug delivery device, or the like) using a wired or wireless link, or may be calculated iteratively by the controller 102 or controller 109, such as every 5 minutes.

Although not shown, in some embodiments, the sensor 108 may include a processor, memory, a sensing or measuring device, and a communication device. The memory may store an instance of an AP application as well as other programming code and be operable to store data related to the AP application.

In various embodiments, the sensing/measuring device of the sensor 108 may include one or more sensing elements, such as a blood glucose measurement element, a heart rate monitor, a blood oxygen sensor element, or the like. The sensor processor may include discrete, specialized logic and/or components, an application-specific integrated circuit, a microcontroller or processor that executes software instructions, firmware, programming instructions stored in memory, or any combination thereof.

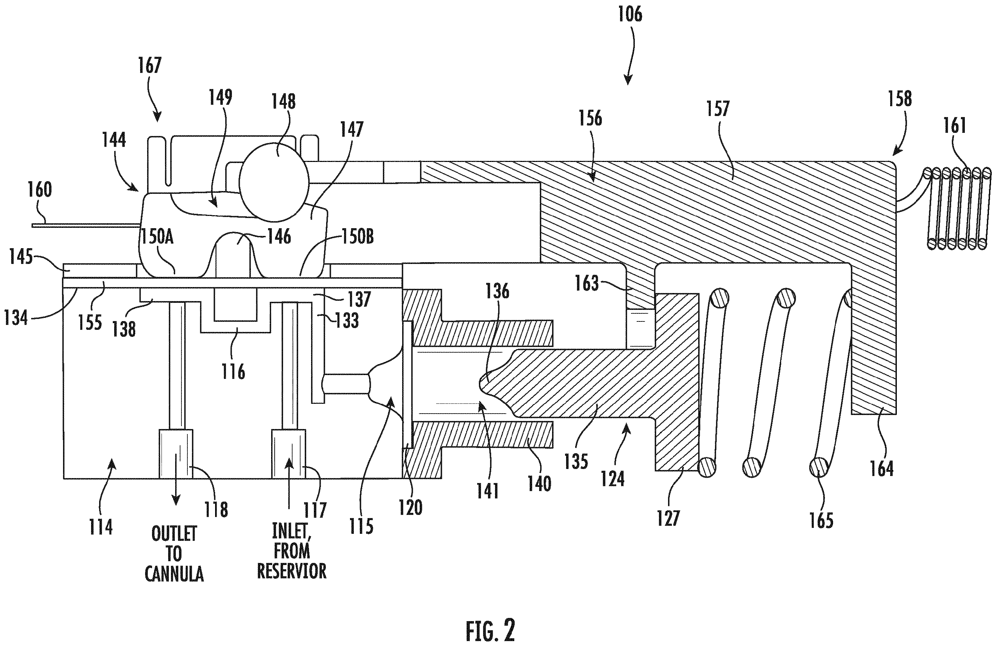

Turning now to **FIG. 2****,** the drive mechanism 106 according to embodiments of the present disclosure will be described in greater detail. As shown, the drive mechanism 106 may include the housing 114 defining the pump chamber 115, the channel chamber 116, the inlet channel 117, and the outlet channel 118. The pump chamber 115 and the channel chamber 116 may be fluidly connected by an internal channel 133. Although non-limiting, the pump chamber 115 may have a bell shape/profile, which compliments the exterior shape/profile of a tip 136 of the plunger 124. The channel chamber 116 may fluidly connect the inlet channel 117 with the outlet channel 118. More specifically, the inlet channel 117 may include an inlet chamber 137 and the outlet channel 118 may include an outlet chamber 138, wherein the channel chamber 116 extends between the inlet chamber 137 and the outlet chamber 138. Although non-limiting, the channel chamber 116 may have a U-shaped profile, as shown, or a substantially straight/horizontal profile.

Coupled to the housing 114, or integrally formed therewith, may be a plunger cylinder 140 defining an internal plunger chamber 141 configured to receive a shaft 135 of the plunger 124. Although not limited to any particular shape or configuration, the plunger 124 may include the tip 136 at a first end and a flange 127 at a second end.

As further shown, the resilient sealing member 120 may be positioned across the pump chamber 115, creating a liquid-tight seal between the internal plunger chamber 141 and the pump chamber 115. In some embodiments, the resilient sealing member 120 is sandwiched between the housing 114 and the plunger cylinder 140. Although non-limiting, the resilient sealing member 120 may be made from a shape memory polymer, such as a polymeric smart material, which has the ability to return from a temporary deformed/compressed shape to a permanent or natural non-deformed/un-compressed shape. For example, the straight/vertical configuration of the resilient sealing member 120 may correspond to its natural or permanent shape.

The drive mechanism 106 may further include a switching device 144 coupled to the housing 114. In some embodiments, the switching device 144 may be a mechanical toggle including a base 145 and a rocker 147 operable to rotate about a fulcrum 146 of the base 145 based on a position of a ball 148 within a slot 149. As shown, the rocker 147 may include a pair of legs 150A, 150B straddling the fulcrum 146 and extending into an opening in the base 145. During operation, one of the legs 150A, 150B of the rocker 147 may be pressed against a second resilient sealing member 155, depending on the position of the rocker 147, to control flow of the liquid drug through the inlet and outlet channels 117, 118. As shown, the second resilient sealing member 155 may extend across the inlet chamber 137 and the outlet chamber 138, thus allowing the inlet channel 117, the outlet channel 118, and the internal channel 133 to be fluidly connected by the channel chamber 116. In some embodiments, the second resilient sealing member 155 may be sandwiched between the housing 114 and the base 145 of the switching device 144. Although non-limiting, the second resilient sealing member 155 may be made from a shape memory polymer, such as a polymeric smart material, which has the ability to return from a temporary deformed/compressed shape to a permanent or natural non-deformed/uncompressed shape. For example, the straight/horizontal configuration of the second resilient sealing member 155 may correspond to its natural or permanent shape.

The drive mechanism 106 may further include a follower 156 coupled to the switching device 144 and to the plunger 124. As shown, the follower 156 may include a body 157 having a first end 167 opposite a second end 158. Coupled to the first end 167 of the body 157 may be the first biasing device 160, (e.g., a SMA wire, motor with lead screw, linear actuator, etc.), and coupled to the second end 158 of the body 157 may be a second biasing device 161 (e.g., return spring). The first biasing device 160 may cause the follower 156 to move in a first direction and the second biasing device 161 may cause the follower 156 to move in a second direction, opposite the first direction.

In a case where the first biasing device 160 is an SMA wire, the power element 132 (FIG. 1) may be activated to energize the SMA wire, which causes the SMA wire to change shape (e.g., contract). More specifically, the activated SMA wire begins to shorten, after having previously been passively relaxed, pulling the follower 156 and the plunger 124 towards the housing 114 as the SMA wire strives to return to its memorized or natural/pre-stressed shape and length. In various embodiments, contraction of the SMA wire may be controlled by increasing or decreasing heat generated by the power element 132. For example, a lower current supplied to the SMA wire may cause the follower 156 to move more slowly than a higher current.

Although non-limiting, the SMA wire may be linear or may generally be V-shaped or U-shaped, with one end of the SMA wire, or a base of the SMA wire (i.e., the area where both legs meet) coupled to the first end 167 of the follower 156. In the latter case, the total force exerted by each leg may be summed, with the total pulling force accordingly doubled for a U or V-shaped arrangement. For a given required total force, thinner SMA wires may accordingly be used, with the electric resistance increasing with decreasing diameter. Further, since the electric resistance depends on the total length of the SMA wire, the electric resistances of a U- or V-shaped arrangement is accordingly double the electric resistance of a single leg of identical diameter. The double leg configuration of the SMA wire accordingly results in a comparatively high electric resistance, which is favorable in order to limit the required current for heating. In other embodiments, alternative folding arrangements are possible, such as a threefold (resulting in an "N-shape") or a fourfold (resulting in an "M-shape").

In some embodiments, the follower 156 may include a first engagement wall 163 operable to engage the flange 127 of the plunger 124 and a second engagement wall 164 in abutment with a plunger spring 165. As shown, the plunger spring 165 may be compressed between the second engagement wall 164 and the flange 127 of the plunger 124 to bias the plunger 124 towards the first engagement wall 163. The second biasing device 161 may be directly coupled to the second engagement wall 164.

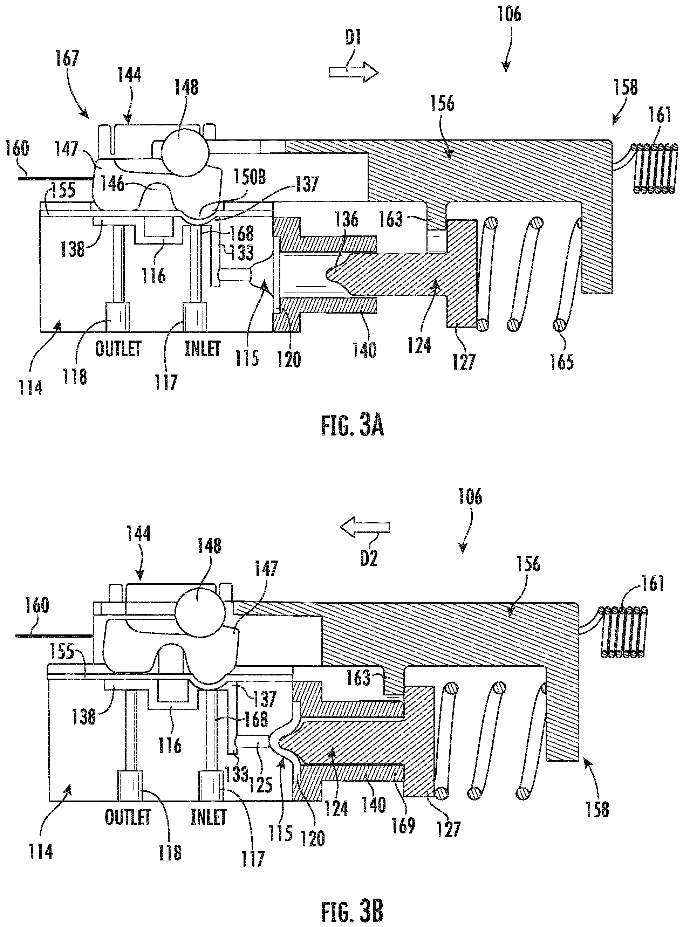

**FIGs. 3A-3D** illustrate the drive mechanism 106 at various stages of a filling and pumping cycle according to embodiments of the present disclosure. Although not demonstrated, the drive mechanism 106 may first be primed to fill the pump chamber 115, e.g., by repeatedly cycling in fluid and expelling air from the inlet channel 117, the pump chamber 115, the channel chamber 116, and the outlet channel 118. At the stage demonstrated in **FIG. 3A****,** the first biasing device 160 (e.g., SMA wire) may be inactive, which allows the follower 156 to move away from the housing 114, e.g., along direction 'D1,' in response to a force from the second biasing device 161. Movement of the follower 156 may cause the ball 148 of the switching device 144 to roll towards the plunger 124, which in turn may cause the rocker 147 to rotate (e.g., clockwise) about the fulcrum 146 and the leg 150B of the rocker 147 to engage the second resilient sealing member 155. Due to the force from the leg 150B, the second resilient sealing member 155 may be deformed or depressed into the inlet chamber 137 and against an entrance 168 of the inlet channel 117. A seal is created therebetween to prevent further flow of the liquid drug 125 from the inlet channel 117.

Furthermore, movement of the follower 156 may cause the first engagement wall 163 to engage the flange 127 of the plunger 124, which in turn may cause the plunger 124 to be retracted relative to the pump chamber 115 and the resilient sealing member 120. A force from the plunger spring 165 may maintain contact between the first engagement wall 163 and the flange 127 of the plunger 124 as the plunger 124 moves along direction 'D1'. An amount of the liquid drug 125 within the pump chamber 115 may change as the resilient sealing member 120 changes configuration. For example, in response to movement by the resilient sealing member 120 into the flat/vertical position shown, the liquid drug 125 may be drawn through the inlet channel 117 and into the pump chamber 115. More specifically, negative pressure created in the pump chamber 115 by the reconfiguration of the resilient sealing member 120 and the plunger 124 causes the liquid drug 125 to flow through the internal channel 133 and into the pump chamber 115 while the inlet channel 117 remains closed at the entrance 168.

As demonstrated in **FIG. 3B****,** the first biasing device 160 may be activated, e.g., in response to a current received from the power element 132 **(****FIG. 1****),** which causes the follower 156 to move towards the housing 114, e.g., along direction 'D2,' overcoming the force of the second biasing device 161. The follower 156 and the plunger 124 may move towards the housing 114 until the flange 127 of the plunger 124 abuts an end 169 of the plunger cylinder 140 and the tip 136 of the plunger 124 is inserted into the pump chamber 115. With the entrance 168 to the inlet channel 117 closed by the second resilient sealing member 155, the liquid drug 125 is displaced from the pump chamber 115 and the internal channel 133, and forced towards the channel chamber 116 for delivery to the patient via the outlet chamber 138 and the outlet channel 118.

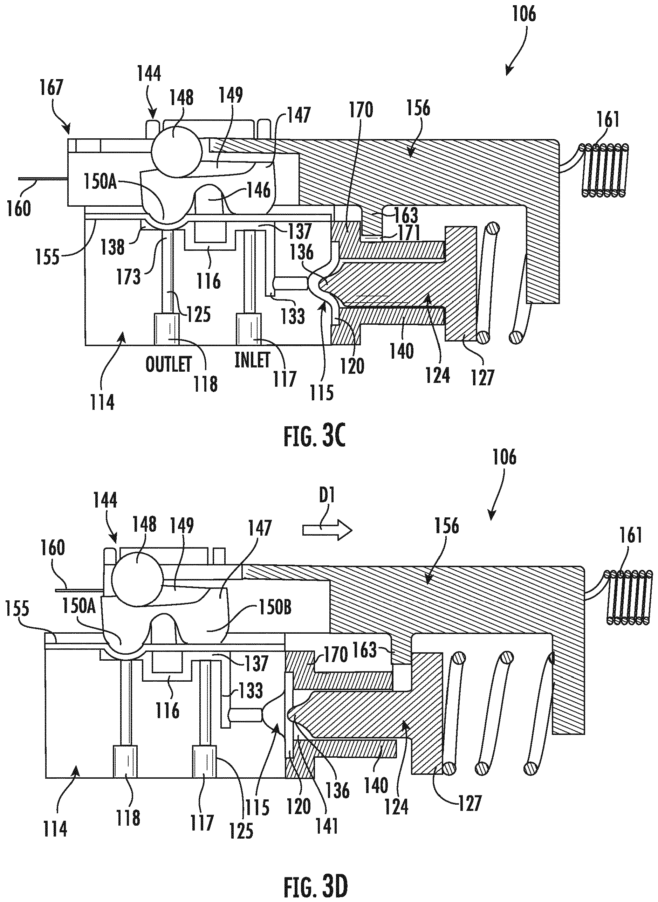

As demonstrated in **FIG. 3C****,** activation of the first biasing device 160 continues until the first engagement wall 163 of the follower 156 separates from the flange 127 of the plunger 124 and moves into contact with a plunger wall flange 170 of the plunger cylinder 140. In some embodiments, the first engagement wall 163 may have an opening or recess 171, which allows the first engagement wall 163 to pass over an exterior of the plunger cylinder 140. Movement of the follower 156 into the configuration shown causes the switching device 144 to transition from a first position (shown in **FIGs. 3A-3B****)** to a second position in which leg 150A of the rocker 147 depresses the second resilient sealing member 155 into the outlet chamber 138 and against an entrance 173 of the outlet channel 118. More specifically, the ball 148 may move within the slot 149 towards the first end 167 of the follower 156 until the rocker 147 rotates (e.g., counterclockwise) about the fulcrum 146. As a result, a liquid-tight seal is created across the entrance 173 of the outlet channel 118 to prevent further flow of the liquid drug 125 from the channel chamber 116 to the outlet channel 118.

In some embodiments, deactivation of the SMA wire of the first biasing device 160, e.g., by the controller 102 **(****FIG. 1****),** may occur following expiration of a predetermined time period triggered by the initial activation of the SMA wire. The predetermined time period may be sufficient to allow a controlled dose (e.g., .05 mL or .025 mL) of the liquid drug 125 to enter and then be discharged from the pump chamber 115, and then delivered to the patient 103 via the cannula 129.

As demonstrated in **FIG. 3D****,** the second biasing device 161 may pull the follower 156 away from the housing 114, e.g., along direction 'D1,' which causes the first engagement wall 163 to move from the plunger wall flange 170 of the plunger cylinder 140 to the flange 127 of the plunger 124. In some embodiments, when the first biasing device 160 is an SMA wire, current to the SMA wire is decreased or discontinued to relax/elongate the SMA wire, which allows the force of the second biasing device 161 pull the follower 156 along direction 'D1.' As shown, a force on the flange 127 from the first engagement wall 163 may cause the tip 136 of the plunger 124 to retract from the pump chamber 115. Negative pressure created as the plunger 124 is retracted from the internal plunger chamber 141 causes the liquid drug 125 to be drawn through the inlet channel 117 again. More specifically, an amount of the liquid drug 125 within the pump chamber 115 may change as the resilient sealing member 120 changes configuration. For example, in response to movement by the resilient sealing member 120 into the flat/vertical position shown, the liquid drug will be drawn through the inlet channel 117 and into the pump chamber 115 via the inlet chamber 137 and the internal channel 133.

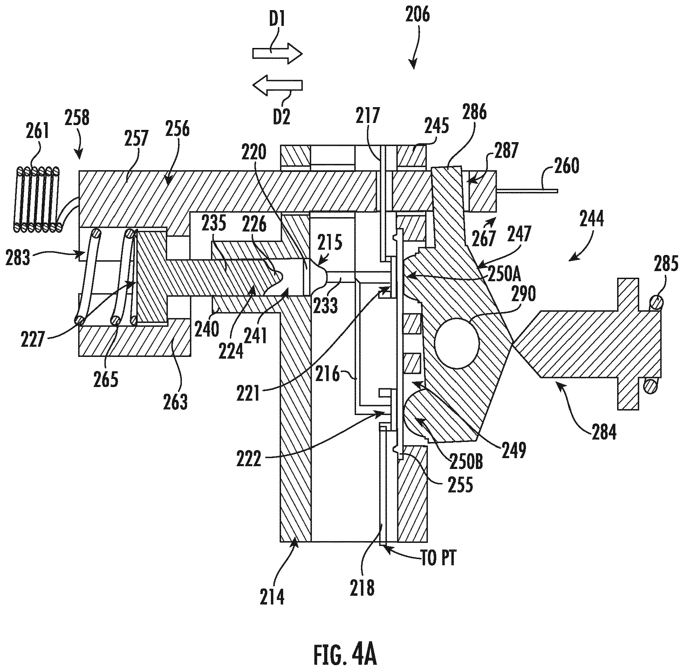

**FIGs. 4A-4C** illustrate an alternative drive mechanism 206 according to embodiments of the present disclosure. The drive mechanism 206 may be part of a delivery pump device, such as delivery pump device 105 of **FIG. 1****.** As shown, the drive mechanism 206 may include a housing 214 defining a pump chamber 215, a channel chamber or pathway 216, an inlet channel 217 fluidly connected with a reservoir (not shown), and an outlet channel 218 for delivering a liquid drug to a patient, e.g., via a cannula. The inlet channel 217 may be connected with an inlet valve 221, and the outlet channel 218 may be connected with an outlet valve 222. In some embodiments, the pump chamber 215 and the second pathway 216 may be fluidly connected by an internal channel 233 through the housing 214. Although non-limiting, the pump chamber 215 may have a bell shape/profile to receive a correspondingly shaped tip 226 of a plunger 224.

As shown, the housing 214 may include a plunger cylinder 240 defining an internal plunger chamber 241 configured to receive a shaft 235 of the plunger 224. The plunger 224 may further include a flange 227 in contact with a plunger spring 265. The flange 227 and the plunger spring 265 may be positioned within a cavity 283 of a follower 256, wherein the follower 256 is coupled to the housing 214. In some embodiments, an engagement wall 263 of the follower 256 may be in contact with the flange 227 of the plunger 224.

As further shown, the drive mechanism 206 may include a resilient sealing member 220 positioned across the pump chamber 215, creating a liquid-tight seal between the internal plunger chamber 241 and the pump chamber 215. In some embodiments, the resilient sealing member 220 is directly coupled to the housing 214. Although non-limiting, the resilient sealing member 220 may be made from a shape memory polymer, such as a polymeric smart material, which has the ability to return from a temporary deformed/compressed shape to a permanent or natural, undeformed shape. For example, the straight/vertical configuration of the resilient sealing member 220 may correspond to its natural or permanent shape.

The drive mechanism 206 may further include a switching device 244 coupled to the housing 214. In some embodiments, the switching device 244 may be a mechanical toggle including a base 245, a rocker 247, and a bias 284. In some embodiments, the bias 284 is loaded by a spring 285. As shown, the rocker 247 may include a pair of legs 250A, 250B extending into corresponding openings 249 of the base 245. During operation, one of the legs 250A, 250B of the rocker 247 may be pressed against a second resilient sealing member 255 depending on the position of the rocker 247. Although non-limiting, the second resilient sealing member 155 may be made from a shape memory polymer, such as a polymeric smart material, which has the ability to return from a temporary deformed/compressed shape to a permanent or natural, undeformed shape. For example, the straight/horizontal configuration of the second resilient sealing member 255 may correspond to its natural or permanent shape.

As shown, an arm 286 of the rocker 247 may extend through an opening 287 of the follower 256. The follower 256 may include a body 257 having a first end 267 opposite a second end 258. Coupled to the first end 267 may be a first biasing device 260, (e.g., an SMA wire, motor with lead screw, or linear actuator), and coupled to the second end 258 may be a second biasing device 261 (e.g., a return spring). The first biasing device 260 may cause the follower 256 to move in a first direction 'D1', and the second biasing device 261 may cause the follower 256 to move in a second direction 'D2', opposite the first direction.

In the case the first biasing device 260 is an SMA wire, a power element (e.g., power element 132 in **FIG. 1****)** may be activated to energize the SMA wire, which causes the SMA wire to change shape (e.g., contract). More specifically, the activated SMA wire begins to shorten, after having previously been passively relaxed, pulling the follower 256 and the plunger 224 along direction 'D1' as the SMA wire strives to return to its memorized or natural/pre-stressed shape and length. In various embodiments, contraction of the SMA wire may be controlled by increasing or decreasing heat generated by the power element. For example, a lower current supplied to the SMA wire may cause the follower 256 to move more slowly than a higher current.

At the stage demonstrated in **FIG. 4A****,** the first biasing device 260 may be inactive, which allows the second biasing device 261 to pull the follower 256 along direction 'D2,' thus causing the rocker 247 to rotate (e.g. counterclockwise) about a center pin 290. Due to the force from the bias 284 on the leg 250A, the second resilient sealing member 255 may be deformed or depressed against the inlet valve 221 to create a liquid-tight seal therebetween, which prevents further flow of a liquid drug from the inlet channel 217. As further shown, the plunger 224 is retracted relative to the pump chamber 215 and the resilient sealing member 220, which may cause the liquid drug to fill the pump chamber 215. A force from the plunger spring 265 may maintain contact between the engagement wall 263 of the follower 256 and the flange 227 of the plunger 224 as the plunger 224 moves out of the plunger cylinder 240.

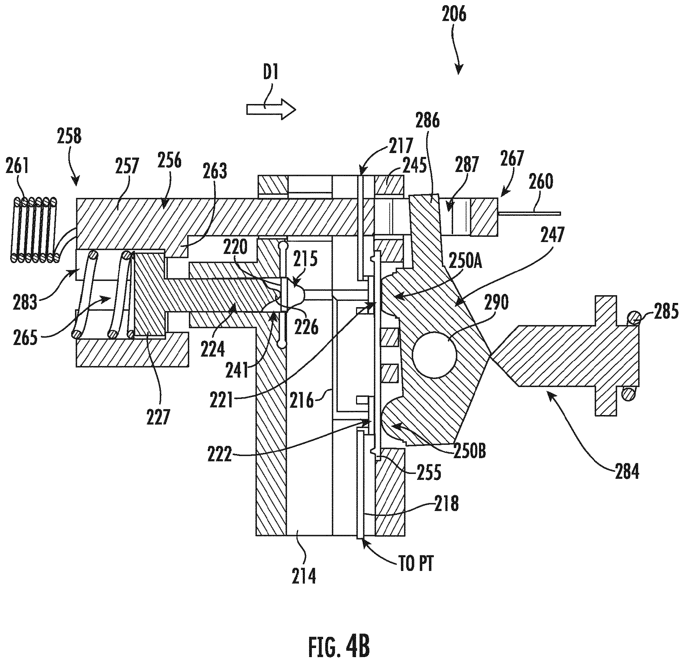

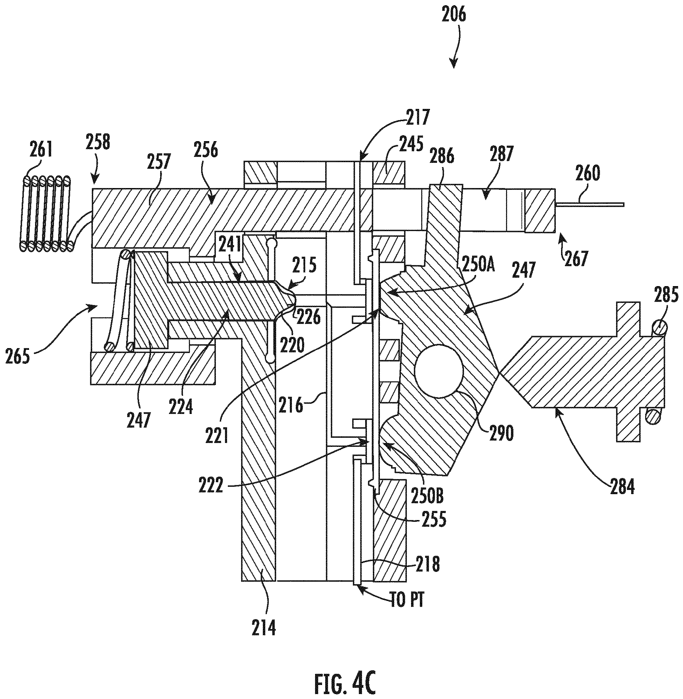

As demonstrated in **FIG. 4B****,** the first biasing device 260 may be activated, e.g., in response to a current received from the power element, which causes the follower 256 to start moving along direction 'D1'. The plunger 224 moves together with the follower 256, causing the plunger 224 to travel within the internal plunger chamber 241 and towards the resilient sealing member 220. Activation of the first biasing device 260 continues, as demonstrated in **FIG. 4C****,** which causes the tip 226 of the plunger 224 and the resilient sealing member 220 to fully enter the pump chamber 215 and expel the liquid drug therefrom. A force from the plunger 224 may cause the liquid drug to travel through the second pathway 216, through the outlet valve 222, and out through the outlet channel 218. As the rocker 247 is further rotated by the follower 256, the inlet valve 221 is opened and the outlet valve 222 is closed, thus preventing the liquid from exiting through the outlet channel 218.

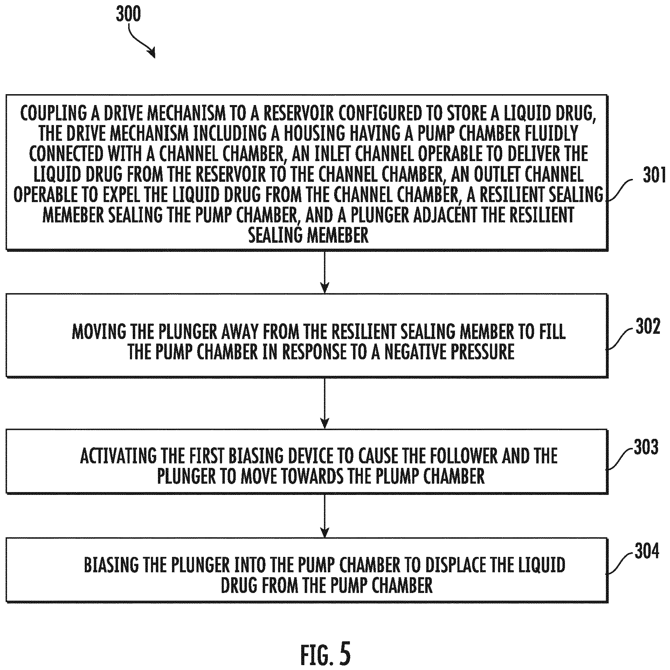

**FIG. 5** illustrates an example process 300 according to embodiments of the present disclosure. At block 301, the process 300 may include coupling a drive mechanism to a reservoir configured to store a liquid drug. In some embodiments, the drive mechanism is part of a wearable drug delivery device. The drive mechanism may include a housing having a pump chamber fluidly connected with a channel chamber, an inlet channel operable to deliver the liquid drug from the reservoir to the channel chamber, and an outlet channel operable to expel the liquid drug from the channel chamber. The drive mechanism may further include a resilient sealing member sealing the pump chamber, and a plunger adjacent the resilient sealing member. In some embodiments, the resilient sealing member are directly attached/coupled to one another.

At block 302, the process 300 may include moving the plunger away from the resilient sealing member to fill the pump chamber, while the outlet channel is closed, in response to a negative pressure created within the pump chamber. In some embodiments, the plunger is coupled to a follower, which is biasable in a first direction by a first biasing device and in a second direction by a second biasing device. In some embodiments, the first biasing device is an SMA wire, wherein deactivating the SMA wire may cause the follower and the plunger to move away from the housing.

At block 303, the process 300 may further include activating the first biasing device to cause the follower and the plunger to move towards the pump chamber. In some embodiments, a mechanical rocker device transitions from a first position in which a second resilient sealing member is biased against the outlet channel, to a second position in which the second resilient sealing member is biased against the inlet channel. In some embodiments, the follower is coupled to the mechanical rocker device, wherein movement of the follower relative to the housing causes the mechanical rocker device to transition between the first and second positions.

At block 304, the process 300 may further include biasing the plunger into the pump chamber to displace the liquid drug from the pump chamber. In some embodiments, the liquid drug is forced from the pump chamber, through an internal channel of the housing and the channel chamber, and out through the outlet channel for delivery to patient, e.g., via a cannula. The SMA wire may then be de-activated again to move the plunger and the resilient sealing member from within the pump chamber to a neutral/natural position, to draw in more liquid drug, thus repeating the cycle. In some embodiments, deactivation of the SMA wire, e.g., by a controller, may occur following expiration of a predetermined time period triggered by the initial activation of the SMA wire. The predetermined time period may be sufficient to allow a controlled dose (e.g., 0.05 mL or 0.025 mL) of the liquid drug to enter and then be extinguished from the pump chamber. Alternatively, deactivation may be triggered when the resilient sealing member reaches a particular position within the pump chamber, or when the plunger reaches a particular position within the plunger chamber. For example, electrodes may be placed within a flange of the plunger and in the end of the plunger cylinder to determine a relative position of the flange, which may cause the controller of the drug delivery device to take a certain action, such as de-activation of the SMA wire.

As used herein, the algorithms or computer applications that manage blood glucose levels and insulin therapy may be referred to as an "artificial pancreas" algorithm-based system, or more generally, an artificial pancreas (AP) application. An AP application may be programming code stored in a memory device and that is executable by a processor, controller or computer device.

The techniques described herein for a drug delivery system (e.g., the system 100 or any components thereof) may be implemented in hardware, software, or any combination thereof. Any component as described herein may be implemented in hardware, software, or any combination thereof. For example, the system 100 or any components thereof may be implemented in hardware, software, or any combination thereof. Software related implementations of the techniques described herein may include, but are not limited to, firmware, application specific software, or any other type of computer readable instructions that may be executed by one or more processors. Hardware related implementations of the techniques described herein may include, but are not limited to, integrated circuits (ICs), application specific ICs (ASICs), field programmable arrays (FPGAs), and/or programmable logic devices (PLDs). In some examples, the techniques described herein, and/or any system or constituent component described herein may be implemented with a processor executing computer readable instructions stored on one or more memory components.

Some examples of the disclosed devices may be implemented, for example, using a storage medium, a computer-readable medium, or an article of manufacture which may store an instruction or a set of instructions that, if executed by a machine (i.e., processor or controller), may cause the machine to perform a method and/or operation in accordance with examples of the disclosure. Such a machine may include, for example, any suitable processing platform, computing platform, computing device, processing device, computing system, processing system, computer, processor, or the like, and may be implemented using any suitable combination of hardware and/or software. The computer-readable medium or article may include, for example, any suitable type of memory unit, memory, memory article, memory medium, storage device, storage article, storage medium and/or storage unit, for example, memory (including non-transitory memory), removable or non-removable media, erasable or non-erasable media, writeable or re-writeable media, digital or analog media, hard disk, floppy disk, Compact Disk Read Only Memory (CD-ROM), Compact Disk Recordable (CD-R), Compact Disk Rewriteable (CD-RW), optical disk, magnetic media, magneto-optical media, removable memory cards or disks, various types of Digital Versatile Disk (DVD), a tape, a cassette, or the like. The instructions may include any suitable type of code, such as source code, compiled code, interpreted code, executable code, static code, dynamic code, encrypted code, programming code, and the like, implemented using any suitable high-level, low-level, object-oriented, visual, compiled and/or interpreted programming language. The non-transitory computer readable medium embodied programming code may cause a processor when executing the programming code to perform functions, such as those described herein.

Certain examples of the present disclosed subject matter were described above. It is, however, expressly noted that the present disclosed subject matter is not limited to those examples, but rather the intention is that additions and modifications to what was expressly described herein are also included within the scope of the disclosed subject matter. Moreover, it is to be understood that the features of the various examples described herein were not mutually exclusive and may exist in various combinations and permutations, even if such combinations or permutations were not made express herein, without departing from the spirit and scope of the disclosed subject matter. In fact, variations, modifications, and other implementations of what was described herein will occur to those of ordinary skill in the art without departing from the spirit and the scope of the disclosed subject matter. As such, the disclosed subject matter is not to be defined only by the preceding illustrative description.

Program aspects of the technology may be thought of as "products" or "articles of manufacture" typically in the form of executable code and/or associated data that is carried on or embodied in a type of machine readable medium. Storage type media include any or all of the tangible memory of the computers, processors or the like, or associated modules thereof, such as various semiconductor memories, tape drives, disk drives and the like, which may provide non-transitory storage at any time for the software programming. It is emphasized that the Abstract of the Disclosure is provided to allow a reader to quickly ascertain the nature of the technical disclosure. It is submitted with the understanding that it will not be used to interpret or limit the scope or meaning of the claims. In addition, in the foregoing Detailed Description, various features are grouped together in a single example for streamlining the disclosure. This method of disclosure is not to be interpreted as reflecting an intention that the claimed examples require more features than are expressly recited in each claim. Rather, as the following claims reflect, inventive subject matter lies in less than all features of a single disclosed example. Thus, the following claims are hereby incorporated into the Detailed Description, with each claim standing on its own as a separate example. In the appended claims, the terms "including" and "in which" are used as the plain-English equivalents of the respective terms "comprising" and "wherein," respectively. Moreover, the terms "first," "second," "third," and so forth, are used merely as labels and are not intended to impose numerical requirements on their objects.

The foregoing description of example examples has been presented for the purposes of illustration and description. It is not intended to be exhaustive or to limit the present disclosure to the precise forms disclosed. Many modifications and variations are possible in light of this disclosure. It is intended that the scope of the present disclosure be limited not by this detailed description, but rather by the claims appended hereto. Future filed applications claiming priority to this application may claim the disclosed subject matter in a different manner and may generally include any set of one or more limitations as variously disclosed or otherwise demonstrated herein.

## Claims

1. A drive mechanism (106) of a wearable drug delivery device (100), the drive mechanism (106) comprising:
a housing (114) comprising:
a pump chamber (115) fluidly connected with a channel chamber (116);
an inlet channel (117) operable to deliver a liquid drug from a reservoir of the wearable drug delivery device to the channel chamber (116); and
an outlet channel (118) operable to expel the liquid drug from the channel chamber (116);
a resilient sealing member (120) sealing the pump chamber (115); and
a plunger (124) adjacent the resilient sealing member (120), wherein the plunger (124) is operable to enter the pump chamber (115) to expel the liquid drug from the pump chamber (115);
and a second resilient sealing member (155) positioned within the channel chamber (116),
wherein the second resilient sealing member (155) is operable to seal the inlet channel (117) and the outlet channel (118);
**characterised in that**
the drive mechanism (106) further comprises
a mechanical rocker device (147), wherein in a first position the mechanical rocker device (147) biases the second resilient sealing member (155) against an entrance (168) of the inlet channel (117), and wherein in a second position the mechanical rocker device (147) biases the second resilient sealing member (155) against an entrance (173) of the outlet channel (118).

2. The drive mechanism (106) of claim 1, further comprising:
a follower (156) coupled to the mechanical rocker device (147), wherein movement of the follower (156) relative to the housing (114) causes the mechanical rocker device (147) to move between the first and second positions; and
a shape memory alloy wire coupled to the follower (156), wherein the shape memory alloy wire causes the follower (156) to move relative to the housing (114).

3. The wearable drug delivery device (100) of claim 2, further comprising a power source coupled to the shape memory alloy wire, wherein power from the power source causes the shape memory wire (160) to contract.

4. The wearable drug delivery device (100) of claim 3, further comprising a controller communicatively coupled to the power source, wherein the controller is operable to:
receive an input indicating an automated insulin delivery (AID) application setting; and
in response to the input, activate the power source.

5. The drive mechanism (106) of claim 2, further comprising a return spring coupled to the follower (156), wherein the biasing device causes the follower (156) to move in a first direction, and wherein the return spring causes the follower (156) to move in a second direction, opposite the first direction.

6. The wearable drug delivery device (100) of claim 2, further comprising a plunger spring (165) in abutment with the plunger (124) and with the follower (156), wherein the plunger spring (165) is operable to cause the plunger (124) to enter the pump chamber (115) as the follower (156) moves towards the housing (114).

7. The drive mechanism (106) of any of claims 1-6, wherein the wearable drug delivery device (100) further comprises a reservoir configured to store the liquid drug and a delivery pump device that includes the drive mechanism (106).

8. The wearable drug delivery device (100) of any of claims 1-7, wherein the plunger (124) is positioned within a plunger chamber (241), and wherein the resilient sealing member (120) is positioned between the plunger chamber (241) and the pump chamber (115).

## Patentansprüche

1. Antriebsmechanismus (106) einer tragbaren Medikamentenverabreichungsvorrichtung (100), wobei der Antriebsmechanismus (106) Folgendes umfasst:
ein Gehäuse (114), das Folgendes umfasst:
eine Pumpenkammer (115), die fluidisch mit einer Kanalkammer (116) verbunden ist,
einen Einlasskanal (117), der dahingehend betätigbar ist, der Kanalkammer (116) ein flüssiges Medikament aus einem Reservoir der tragbaren
Medikamentenverabreichungsvorrichtung zuzuführen, und
einen Auslasskanal (118), der dahingehend betätigbar ist, das flüssige Medikament aus der Kanalkammer (116) auszustoßen,
ein elastisches Dichtungselement (120), das die Pumpenkammer (115) abdichtet, und
einen dem elastischen Dichtungselement (120) benachbarten Kolben (124), wobei der Kolben (124) dahingehend betätigbar ist, in die Pumpenkammer (115) einzutreten, um das flüssige Medikament aus der Pumpenkammer (115) auszustoßen,
und ein zweites elastisches Dichtungselement (155), das in der Kanalkammer (116) positioniert ist, wobei das zweite elastische Dichtungselement (155) dahingehend betätigbar ist, den Einlasskanal (117) und den Auslasskanal (118) abzudichten,
**dadurch gekennzeichnet, dass**
der Antriebsmechanismus (106) ferner eine mechanische Wippvorrichtung (147) umfasst,
wobei die mechanische Wippvorrichtung (147) in einer ersten Position das zweite elastische Dichtungselement (155) gegen einen Eingang (168) des Einlasskanals (117) vorspannt und
wobei die mechanische Wippvorrichtung (147) in einer zweiten Position das zweite elastische Dichtungselement (155) gegen einen Eingang (173) des Auslasskanals (118) vorspannt.

2. Antriebsmechanismus (106) nach Anspruch 1, ferner umfassend:
einen Mitnehmer (156), der an die mechanische Wippvorrichtung (147) gekoppelt ist, wobei durch eine Bewegung des Mitnehmers (156) bezüglich des Gehäuses (114) veranlasst wird,
dass sich die mechanische Wippvorrichtung (147) zwischen der ersten und der zweiten Position bewegt, und
einen Formgedächtnislegierungsdraht, der an den Mitnehmer (156) gekoppelt ist, wobei der Formgedächtnislegierungsdraht veranlasst, dass sich der Mitnehmer (156) bezüglich des Gehäuses (114) bewegt.

3. Tragbare Medikamentenverabreichungsvorrichtung (100) nach Anspruch 2, ferner umfassend eine Energiequelle, die an den Formgedächtnislegierungsdraht gekoppelt ist, wobei durch Energie von der Energiequelle veranlasst wird, dass sich der Formgedächtnisdraht (160) zusammenzieht.

4. Tragbare Medikamentenverabreichungsvorrichtung (100) nach Anspruch 3, ferner umfassend eine Steuerung, die kommunikativ an die Energiequelle gekoppelt ist, wobei die Steuerung dahingehend betätigbar ist,
eine Eingabe, die eine Anwendungseinstellung zur automatischen Insulinverabreichung (AID) angibt, zu empfangen und
als Reaktion auf die Eingabe die Energiequelle zu aktivieren.

5. Antriebsmechanismus (106) nach Anspruch 2, ferner umfassend eine Rückstellfeder, die an den Mitnehmer (156) gekoppelt ist, wobei durch die Vorspannvorrichtung veranlasst wird, dass sich der Mitnehmer (156) in einer ersten Richtung bewegt, und wobei durch die Rückstellfeder veranlasst wird, dass sich der Mitnehmer (156) in einer der ersten Richtung entgegengesetzten zweiten Richtung bewegt.

6. Tragbare Medikamentenverabreichungsvorrichtung (100) nach Anspruch 2, ferner umfassend eine Kolbenfeder (165) in Anlage an dem Kolben (124) und an dem Mitnehmer (156), wobei die Kolbenfeder (165) dahingehend betätigbar ist zu veranlassen, dass der Kolben (124) in die Pumpenkammer (115) eintritt, wenn sich der Mitnehmer (156) zu dem Gehäuse (114) hin bewegt.

7. Antriebsmechanismus (106) nach einem der Ansprüche 1 - 6, wobei die tragbare Medikamentenverabreichungsvorrichtung (100) ferner ein Reservoir, das zum Speichern des flüssigen Medikaments ausgestaltet ist, und eine Verabreichungspumpenvorrichtung, die den Antriebsmechanismus (106) aufweist, umfasst.

8. Tragbare Medikamentenverabreichungsvorrichtung (100) nach einem der Ansprüche 1 - 7, wobei der Kolben (124) in einer Kolbenkammer (241) positioniert ist und wobei das elastische Dichtungselement (120) zwischen der Kolbenkammer (241) und der Pumpenkammer (115) positioniert ist.

## Revendications

1. Mécanisme d'entraînement (106) d'un dispositif portatif d'administration de médicament (100), le mécanisme d'entraînement (106) comprenant :
un logement (114) comprenant :
une chambre de pompe (115) reliée fluidiquement à une chambre de canal (116) ;
un canal d'entrée (117) permettant d'administrer un médicament liquide depuis un réservoir du dispositif portatif d' administration de médicament vers la chambre de canal (116) ; et
un canal de sortie (118) permettant d'expulser le médicament liquide de la chambre de canal (116) ;
un élément d'étanchéité résilient (120) assurant l'étanchéité de la chambre de pompe (115) ; et
un plongeur (124) adjacent à l'élément d'étanchéité résilient (120), dans lequel le plongeur (124) permet d'entrer dans la chambre de pompe (115) pour expulser le médicament liquide de la chambre de pompe (115) ;
et un second élément d'étanchéité résilient (155) positionné à l'intérieur de la chambre de canal (116), dans lequel le second élément d'étanchéité résilient (155) est exploitable pour sceller le canal d'entrée (117) et le canal de sortie (118) ;
**caractérisé en ce que**
le mécanisme d'entraînement (106) comprend en outre un dispositif mécanique à bascule (147), dans lequel, dans une première position, le dispositif mécanique à bascule (147) sollicite le second élément d'étanchéité résilient (155) contre une entrée (168) du canal d'entrée (117), et dans lequel, dans une seconde position, le dispositif mécanique à bascule (147) sollicite le second élément d'étanchéité résilient (155) contre une entrée (173) du canal de sortie (118).

2. Mécanisme d'entraînement (106) selon la revendication 1, comprenant en outre :
un organe suiveur (156) accouplé au dispositif mécanique à bascule (147), dans lequel un mouvement de l'organe suiveur (156) par rapport au logement (114) entraîne le déplacement du dispositif mécanique à bascule (147) entre les première et seconde positions ; et
un fil en alliage à mémoire de forme accouplé à l'organe suiveur (156), dans lequel le fil en alliage à mémoire de forme entraîne le déplacement de l'organe suiveur (156) par rapport au logement (114).

3. Dispositif portatif d'administration de médicament (100) selon la revendication 2, comprenant en outre une source d'alimentation couplée au fil en alliage à mémoire de forme, dans lequel l'alimentation provenant de la source d'alimentation entraîne la contraction du fil à mémoire de forme (160).

4. Dispositif portatif d'administration de médicament (100) selon la revendication 3, comprenant en outre un dispositif de commande couplé en communication à la source d'alimentation, dans lequel le dispositif de commande permet de :
recevoir une entrée indiquant un réglage d'application d'administration automatisée d'insuline (AID) ; et
en réponse à l'entrée, activer la source d'alimentation.

5. Mécanisme d'entraînement (106) selon la revendication 2, comprenant en outre un ressort de rappel accouplé à l'organe suiveur (156), dans lequel le dispositif de sollicitation entraîne le déplacement de l'organe suiveur (156) dans une première direction, et dans lequel le ressort de rappel entraîne le déplacement de l'organe suiveur (156) dans une seconde direction, opposée à la première direction.

6. Dispositif portatif d'administration de médicament (100) selon la revendication 2, comprenant en outre un ressort de plongeur (165) en butée avec le plongeur (124) et avec l'organe suiveur (156), dans lequel le ressort de plongeur (165) permet de faire entrer le plongeur (124) dans la chambre de pompe (115) à mesure que l'organe suiveur (156) se déplace vers le logement (114).

7. Mécanisme d'entraînement (106) selon l'une quelconque des revendications 1 à 6, dans lequel le dispositif portatif d'administration de médicament (100) comprend en outre un réservoir configuré pour stocker le médicament liquide et un dispositif de pompe d'administration qui comporte le mécanisme d'entraînement (106).

8. Dispositif portatif d'administration de médicament (100) selon l'une quelconque des revendications 1 à 7, dans lequel le plongeur (124) est positionné à l'intérieur d'une chambre de plongeur (241), et dans lequel l'élément d'étanchéité résilient (120) est positionné entre la chambre de plongeur (241) et la chambre de pompe (115).
